Europäisches Patentamt

European Patent Office

Office européen des brevets

(11) Publication number: **0 343 640 B1**

# EUROPEAN PATENT SPECIFICATION

(45) Date of publication of patent specification: **12.10.94**

(51) Int. Cl.5: **C07D 213/48**, C07D 307/46, C07D 277/24, C07B 41/06

(21) Application number: **89109401.3**

(22) Date of filing: **24.05.89**

The file contains technical information submitted after the application was filed and not included in this specification

(54) **Process for producing heterocyclic aldehydes.**

(30) Priority: **25.05.88 JP 127900/88**

(43) Date of publication of application:
**29.11.89 Bulletin 89/48**

(45) Publication of the grant of the patent:
**12.10.94 Bulletin 94/41**

(84) Designated Contracting States:
**BE CH DE FR GB IT LI NL**

(56) References cited:
**US-A- 3 935 265**

**CHEMICAL ABSTRACTS, vol. 99, no. 11, September 12, 1983, Columbus, Ohio, USAFUJISAWA T. et al. "Direct and chemoselective conversion of carboxylic acidsinto aldehydes" page 519, column 1, abstract -no. 87 607u**

**CHEMICAL ABSTRACTS, vol. 82, no. 21, May 26, 1975, Columbus, Ohio, USA MURAKIM. et al. "Selective reduction of car- boxylic acids to aldchydes" page 511,column 1, abstract -no. 138 617f**

(73) Proprietor: **MITSUBISHI KASEI CORPORATION 5-2, Marunouchi 2-chome Chiyoda-ku Tokyo 100 (JP)**

(72) Inventor: **Yokoyama, Toshiharu 2-12-3-5-104, Ogawa Machida-shi Tokyo (JP)**
Inventor: **Setoyama, Toru 2-10-4, Ogawa Machida-shi Tokyo (JP)**
Inventor: **Matsuyama, Naoko 15-12-A201, Sumiyoshidai Midori-ku Yokohama-shi Kanagawa (JP)**
Inventor: **Maki, Takao 11-6, Kugenumakaigan 7-chome Fujisawa-shi Kanagawa (JP)**

(74) Representative: **VOSSIUS & PARTNER Postfach 86 07 67 D-81634 München (DE)**

**Description**

This invention relates to a process for producing heterocyclic aldehydes and, more particularly, to a novel process for producing heterocyclic aldehydes useful as intermediates in organic syntheses in high yield.

Heterocyclic aldehydes containing a nitrogen, sulfur or oxygen atom in the ring thereof as a constituting element are extremely useful as intermediates in various organic syntheses. A number of processes for producing the heterocyclic aldehydes have hitherto been proposed.

The most commonly employed of the conventional processes is so-called Rosenmund reduction which comprises catalytic reduction of an acid chloride. This process has a disadvantage of being costly.

Several attempts have been reported, in which a carboxylic acid ester is converted to the corresponding aldehyde through an alcohol. This process however requires severe reaction conditions, incurs high cost, and results in low yields.

Direct reduction of carboxylic acids with molecular hydrogen would be the most advantageous process for the production of aldehydes, but it has been regarded extremely difficult to directly reduce heterocyclic carboxylic acids with molecular hydrogen.

As the latest technique, U.S. Patent 3,935,265 proposes a process for obtaining aldehydes which comprises gaseous phase hydrogenation of aromatic carboxylic acid esters in the presence of an alumina catalyst. However, the U.S. Patent describes that this process is not suitably applicable to basic heterocyclic compounds such as pyridine.

Thus, it has been conventionally believed to be extremely difficult to obtain heterocyclic aldehydes through direct hydrogenation of the corresponding heterocyclic carboxylic acids containing a nitrogen, sulfur or oxygen atom.

The inventors previously succeeded to develop a catalyst which enables the reduction of aromatic, aliphatic or alicyclic carboxylic acids to the corresponding aldehydes in satisfactory yields. The inventors have further conducted extensive investigations on a process for producing aldehydes by hydrogenation of the corresponding carboxylic acids other than these carboxylic acids, particularly heterocyclic carboxylic acids. It has been thought that such carboxylic acids cannot be hydrogenated in high yields in the presence of a solid catalyst. As a result, we have now found a catalyst exerting excellent effects on reduction of heterocyclic carboxylic acids and thus completed the present invention.

One object of this invention is to provide a process for producing a heterocyclic aldehyde in high yield through direct reduction of the corresponding heterocyclic carboxylic acid with molecular hydrogen.

That is, the present invention relates to a process for producing a heterocyclic aldehyde which comprises hydrogenating a heterocyclic carboxylic acid containing at least one atom selected from nitrogen, sulfur, and oxygen atoms as a constituting element of the heterocyclic ring or a derivative thereof with molecular hydrogen in the presence of a catalyst containing an oxide of at least one element selected from the group consisting of zinc, yttrium, cerium, titanium, zirconium and hafnium.

In the heterocyclic carboxylic acids or derivatives thereof which can be used as a starting material in the present invention, the heterocyclic ring contains at least one nitrogen, oxygen or sulfur atom. Specific examples of the heterocyclic ring include pyrrole, furan, thiophene, oxazole, thiazole, oxazoline, imidazole, imidazoline, pyrazole, pyran, thiopyran, pyridine, quinoline, oxazine, thiazine, pyrimidin, pyrazine, triazine, azepin, and oxepin rings.

These rings may have one or more substituents such as alkyl, alkoxy, phenoxy, and hydroxy groups and halogen atoms. Among these substituents, the alkyl group having 1 to 4 carbon atoms is preferred. The carboxyl group or the group derived therefrom may be in the free form or the ester or acid anhydride form.

The esters preferably include those with alkyl groups having from 1 to about 4 carbon atoms, phenyl esters, and cyclohexyl esters. The esters with alkyl groups having from 1 to 4 carbon atoms are more preferred.

Among these heterocyclic carboxylic acids or derivatives thereof as a starting material of this invention, preferred are those having a pyrrole, furan, thiophene, thiazole, pyran or pyridine ring as a skeleton. Specific examples of such compounds are nicotinic acid, furancarboxylic acids, thiazolecarboxylic acid, and derivatives thereof.

The catalyst which can be used in this invention is an oxide of at least one element selected from the group consisting of zinc, yttrium, cerium, and titanium, zirconium, hafnium, preferably zirconium oxide, hafnium oxide, titanium oxide, yttrium oxide, zinc oxide, or cerium oxide.

Physical properties of the oxide catalysts vary depending on the process of production. In the present invention, preferred are those which are neutral and have a large surface area. The oxide catalysts having such properties can be obtained by firing high purity hydroxide, carbonate, nitrate, acetate, etc. of the element at a temperature of from about 200 to 950°C. The oxide catalysts are easy to obtain especially from the hydroxide of the elements.

Addition of at least one specific element to the above-described zirconium oxide results in improvement of catalytic activity and stability. The elements which can be added to this effect are selected from chromium, manganese, cobalt, zinc, indium, lead, bismuth, rhenium, lanthanum, cerium, praseodymium, neodymium, samarium, lithium, sodium, potassium, cesium, etc., with chromium, manganese, cobalt, zinc, indium, and lead being preferred. Chromium is more preferable from the point of economics and treatment.

Addition of these specific elements to zirconium oxide can be carried out by known techniques, such as co-precipitation, impregnation, adsorption, ion exchange, kneading, and the like. These elements may be added to raw materials for the preparation of zirconium oxide. The amount of the specific elements to be added ranges from about 0.001 to 0.5, preferably from about 0.01 to 0.2, in an atomic ratio with respect to zirconium.

In practical use the oxide catalyst is suitably molded or supported on an appropriate inert carrier in a usual manner.

The hydrogenation with molecular hydrogen is advantageously carried out in a gaseous phase. The recommended reaction temperature is from about 200 to 500°C, particularly from about 250 to 400°C. The reaction can be conducted at normal pressure or under reduced pressure.

In cases where the catalyst is used as a fixed bed catalyst, the starting carboxylic acid or its derivative is fed at a space velocity ranging from about 0.01 to 1 $hr^{-1}$, preferably from about 0.03 to 0.3 $hr^{-1}$, in terms of LHSV (liquid hourly space velocity). The space velocity of hydrogen is in the range of from about 100 to 20,000 $hr^{-1}$, preferably from about 500 to 5,000 $hr^{-1}$, in terms of GHSV (gas hourly space velocity). The hydrogen to be fed may contain small amounts of inert gases such as nitrogen and water vapor.

It is a matter of course that the hydrogenation can be effected not only in a fixed bed system but in other reaction systems.

According to the present invention, the catalyst comprising an oxide of at least one element selected from zinc, yttrium, cerium, titanium, zirconium and hafnium makes it possible to directly hydrogenate heterocyclic carboxylic acids or derivatives thereof, that has been conventionally regarded difficult, to thereby produce the corresponding heterocyclic aldehydes in high yield and at low cost.

Therefore, heterocyclic aldehydes useful as intermediates in various organic syntheses can be produced with industrial and economical advantages.

The present invention is illustrated in greater detail in the following Examples and Comparative Examples, but it should be understood that the present invention is not deemed to be limited thereto. In these examples, all the parts and percents are by weight unless otherwise indicated.

EXAMPLE 1

A commercially available zirconyl hydroxide powder was fired at 600°C for 3 hours under air flow, pelletted, and sifted to prepare a zirconia catalyst having a particle size of 10 to 20 mesh. The resulting catalyst (10 ml) was filled in a pyrex reaction column, and gaseous phase hydrogenation of methyl 3-nicotinate was conducted. The reaction conditions and results are shown in Table 1.

EXAMPLE 2

3.47 g of chromium nitrate nonahydrate were dissolved in 100 ml of deionized water and 25 g of commercially available zirconyl hydroxide ($ZrO_2$ content: 85%) were added to the solution. The mixture was evaporated to dryness and fired at 600°C for 3 hours to obtain an oxide composed of chromium and zirconium at an atomic ratio of 5:100. Using the resulting oxide as a starting material, a catalyst was prepared in the same manner as in Example 1, and gaseous phase hydrogenation of methyl 3-nicotinate was conducted. The reaction conditions and results are shown in Table 1.

EXAMPLES 3 TO 7

Catalysts having compositions shown in Table 1 were prepared in the same manner as in Example 2, except for replacing chromium nitrate with the following metal nitrates. Gaseous phase hydrogenation of methyl 3-nicotinate was conducted in the presence of each of the resulting catalysts.

Example 3: Indium nitrate
Example 4: Lead nitrate
Example 5: Zinc nitrate

Example 6: Manganese nitrate
Example 7: Cobalt nitrate
The reaction conditions and results are shown in Table 1.

EXAMPLE 8

Twenty-five grams of hafnium oxychloride octahydrate were dissolved in 1 ℓ of desalted water, and 10% aqueous ammonia was added dropwise thereto while stirring to adjust the solution to a pH of 7.6. After being allowed to stand for a whole day, the formed precipitate was repeatedly washed with water until no more chloride was detected in the filtrate. The precipitate collected by filtration was dried and fired at 600°C for 3 hours under air flow to obtain a hafnium oxide powder. Using the resulting powder as a starting material, a catalyst was prepared in the same manner as in Example 1, and gaseous phase hydrogenation of methyl 3-nicotinate was conducted using the resulting catalyst. The reaction conditions and results are shown in Table 1.

EXAMPLE 9

A titanium oxide catalyst was prepared in the same manner as in Example 8, except for replacing hafnium oxychoride with titanium sulfate. Gaseous phase hydrogenation of methyl 3-nicotinate was conducted using the resulting catalyst. The reaction conditions and results are shown in Table 1.

EXAMPLE 10

Three grams of yttrium nitrate hexahydrate were dissolved in 50 ml of deionized water, and commercially available α-alumina having a particle size of 10 to 20 mesh was added thereto. The mixture was evaporated to dryness and fired at 600°C for 3 hours to obtain an oxide composed of yttrium-aluminum (20% $Y_2O_3/\alpha$-$Al_2O_3$). Using the resulting oxide as a starting material, a catalyst was prepared in the same manner as in Example 1, and gaseous phase hydrogenation of methyl 3-nicotinate was conducted using the resulting catalyst. The reaction conditions and results are shown in Table 1.

EXAMPLE 11

A cerium oxide catalyst was prepared in the same manner as in Example 1, except for replacing zirconyl hydroxide with cerium hydroxide. Gaseous phase hydrogenation of methyl 3-nicotinate was conducted using the resulting catalyst. The reaction conditions and results are shown in Table 1.

EXAMPLE 12

A zinc oxide catalyst was prepared in the same manner as in Example 8, except for replacing hafnium oxychloride with zinc nitrate. Gaseous phase hydrogenation of methyl 3-nicotinate was conducted using the resulting catalyst. The reaction conditions and results are shown in Table 1.

COMPARATIVE EXAMPLE 1

Gaseous phase hydrogenation of methyl 3-nicotinate was conducted using commercially available γ-alumina as a catalyst. The reaction conditions and results are shown in Table 1.

COMPARATIVE EXAMPLE 2

A magnesium oxide catalyst was prepared in the same manner as in Example 1, except for replacing zirconyl hydroxide with magnesium hydroxide. Gaseous phase hydrogenation of methyl 3-nicotinate was conducted using the resulting catalyst. The reaction conditions and results are shown in Table 1.

COMPARATIVE EXAMPLE 3

A niobium oxide catalyst was prepared in the same manner as in Example 1, except for replacing zirconyl hydroxide with niobic acid. Gaseous phase hydrogenation of methyl 3-nicotinate was conducted using the resulting catalyst. The reaction conditions and results are shown in Table 1.

4

## Table 1

| Example No. | Catalyst (Atomic Ratio) | Reaction Conditions | | | Reaction Results | | |
|---|---|---|---|---|---|---|---|
| | | Hydrogen GHSV ($hr^{-1}$) | MN* LHSV (kg/ℓ·cat·hr) | Temperature (°C) | MN Conversion (%) | NA** Selectivity (%) | NA Space Time Yield (mol/ℓ·cat·hr) |
| Example 1 | $ZrO_2$ | 1250 | 0.08 | 340 | 70.0 | 54.6 | 0.20 |
| " 2 | $Cr-ZrO_2(5:100)$ | 1250 | 0.08 | 340 | 96.4 | 58.8 | 0.29 |
| " 3 | $In-ZrO_2(5:100)$ | 1250 | 0.08 | 320 | 92.7 | 58.0 | 0.24 |
| " 4 | $Pb-ZrO_2(5:100)$ | 1250 | 0.08 | 340 | 95.2 | 45.1 | 0.21 |
| " 5 | $Zn-ZrO_2(5:100)$ | 1250 | 0.08 | 340 | 94.9 | 59.1 | 0.26 |
| " 6 | $Mn-ZrO_2(5:100)$ | 1250 | 0.08 | 340 | 89.2 | 62.2 | 0.24 |
| " 7 | $Co-ZrO_2(5:100)$ | 1250 | 0.08 | 340 | 88.2 | 63.1 | 0.28 |
| " 8 | $HfO_2$ | 2080 | 0.14 | 340 | 49.1 | 48.7 | 0.21 |
| " 9 | $TiO_2$ | 1250 | 0.08 | 330 | 77.6 | 51.6 | 0.20 |
| " 10 | $20\%Y_2O_3/\alpha-Al_2O_3$ | 1250 | 0.08 | 400 | 32.1 | 43.1 | 0.088 |
| " 11 | $CeO_2$ | 1250 | 0.08 | 360 | 62.3 | 81.9 | 0.29 |
| " 12 | $ZnO$ | 1250 | 0.08 | 340 | 43.8 | 31.3 | 0.088 |
| Comparative Example 1 | $\gamma-Al_2O_3$ | 1250 | 0.08 | 340 | 99.1 | ~0 | — |
| " 2 | $MgO$ | 1250 | 0.08 | 380 | 47.1 | ~0 | — |
| " 3 | $Nb_2O_5$ | 1250 | 0.08 | 380 | 57.8 | ~0 | — |

Note:  *:  Methyl 3-nicotinate     **:  3-Nicotinaldehyde

## EXAMPLE 13

Zirconyl hydroxide to which chromium nitrate had been added in the same manner as in Example 2 was evaporated to dryness and fired at 700 °C to obtain an oxide composed of chromium-zirconium (atomic ratio: 5:100). With the oxide used as a starting material, a catalyst was prepared in the same manner as in Example 1. Gaseous phase hydrogenation of methyl 3-nicotinate was conducted using the resulting catalyst. The reaction conditions and results are shown in Table 2.

EXAMPLE 14

Gaseous phase hydrogenation of 3-furancarboxylic acid was conducted using an oxide catalyst composed of chromium zirconium (atomic ratio: 5:100) which was prepared in the same manner as in Example 13. The reaction conditions and results are shown in Table 2.

EXAMPLE 15

Gaseous phase hydrogenation of methyl 4-methylthiazole-5-carboxylate was conducted using an oxide catalyst composed of chromium zirconium (atomic ratio: 5:100) which was prepared in the same manner as in Example 13. The reaction conditions and results are shown in Table 2.

Table 2

| Example No. | Substrate | Reaction Conditions | | | Reaction Results | | |
|---|---|---|---|---|---|---|---|
| | | Hydrogen GHSV (hr$^{-1}$) | Substrate LHSV (kg/ℓ·cat·hr) | Temperature (°C) | Substrate Conversion (%) | Aldehyde Selectivity (%) | Aldehyde Space Time Yield (mol/ℓ·cat·hr) |
| 13 | Methyl 3-nicotinate | 1250 | 0.091 | 360 | 85.7 | 82.9 | 0.47 |
| 14 | 3-furan-carboxylic acid | 1250 | 0.075 | 340 | 62.0 | 51.6 | 0.22 |
| 15 | Methyl 4-methyl-thiazole-5-carboxylate | 760 | 0.080 | 315 | 73.8 | 79.8 | 0.30 |

As is apparent from the results of Tables 1 and 2, according to the present invention, heterocyclic carboxylic acids can be directly reduced with molecular hydrogen by the specific action of the catalyst to produce corresponding heterocyclic aldehydes at high conversion, high selectivity, and high yield.

As compared with these results, decarboxylation proceeds in the presence of general oxide catalysts, such as $\gamma$-alumina, magnesium oxide and niobium oxide as in Comparative Example 1 to 3, failing to produce aldehydes.

7

While the invention has been described in detail and with reference to specific embodiments thereof, it will be apparent to one skilled in the art that various changes and modifications can be made therein without departing from the spirit and scope thereof.

**Claims**

1. A process for producing a heterocyclic aldehyde which comprises hydrogenating a heterocyclic carboxylic acid containing at least one hetero atom selected from nitrogen, sulfur, and oxygen atoms as a constituting element of a heterocyclic ring or a derivative thereof with molecular hydrogen in the presence of a catalyst containing an oxide of at least one element selected from the group consisting of zinc, yttrium, cerium, titanium, zirconium and hafnium.

2. A process as claimed in claim 1, wherein said heterocyclic carboxylic acid or derivative thereof contains a pyrrole, furan, thiophene, thiazole, pyran or pyridine ring.

3. A process as claimed in claim 1, wherein said heterocyclic carboxylic acid or derivative thereof is nicotinic acid, furancarboxylic acid or thiazolecarboxylic acid or a derivative thereof.

4. A process as claimed in claim 3, wherein said derivative of the heterocyclic carboxylic acid is an ester with an alkyl group having from 1 to 4 carbon atoms, phenyl or cyclohexyl group.

5. A process as claimed in claim 1, wherein said catalyst contains zirconium oxide, hafnium oxide, titanium oxide, yttrium oxide, zinc oxide or cerium oxide.

6. A process as claimed in claim 1, wherein said catalyst comprises zirconium oxide and at least one metallic element selected from the group consisting of chromium, manganese, cobalt, zinc, indium, and lead.

7. A process as claimed in claim 3, wherein said catalyst comprises zirconium oxide and at least one metallic element selected from the group consisting of chromium, manganese, cobalt, zinc, indium, and lead.

8. A process as claimed in claim 3, wherein said catalyst comprises zirconium oxide and chromium.

9. A process as claimed in claim 6, wherein said metallic element is present at an atomic ratio of from 0.001 to 0.5 with respect to zirconium.

10. A process as claimed in claim 1, wherein said hydrogenating is carried out at a temperature of from 260 to 400°C while feeding the heterocyclic carboxylic acid or derivative thereof at a liquid hourly space velocity of from 0.01 to 1 $hr^{-1}$ and hydrogen at a gas hourly space velocity of from 100 to 20,000 $hr^{-1}$.

**Patentansprüche**

1. Verfahren zur Herstellung eines heterocyclischen Aldehyds, das die Hydrierung einer heterocyclischen Carbonsäure, die mindestens ein Heteroatom aus der Gruppe Stickstoff-, Schwefel- und Sauerstoffatome als Strukturelement des heterocyclischen Rings enthält, oder eines Derivats davon, mit molekularem Wasserstoff in Gegenwart eines Katalysators umfaßt, der ein Oxid mindestens eines Elements aus der Gruppe Zink, Yttrium, Cer, Titan, Zirkon und Hafnium enthält.

2. Verfahren nach Anspruch 1, wobei die heterocyclische Carbonsäure oder das Derivat davon einen Pyrrol-, Furan-, Thiophen-, Thiazol-, Pyran- oder Pyridinring enthält.

3. Verfahren nach Anspruch 1, wobei die heterocyclische Carbonsäure oder das Derivat davon Nicotinsäure, Furancarbonsäure oder Thiazolcarbonsäure oder ein Derivat davon ist.

4. Verfahren nach Anspruch 3, wobei das Derivat der heterocyclischen Carbonsäure ein Ester mit einer 1 bis 4 Kohlenstoffatome aufweisenden Alkylgruppe, einer Phenyl- oder Cyclohexylgruppe ist.

8

**5.** Verfahren nach Anspruch 1, wobei der Katalysator Zirkonoxid, Hafniumoxid, Titanoxid, Yttriumoxid, Zinkoxid oder Ceroxid enthält.

**6.** Verfahren nach Anspruch 1, wobei der Katalysator Zirkonoxid und mindestens ein metallisches Element aus der Gruppe Chrom, Mangan, Kobalt, Zink, Indium und Blei umfaßt.

**7.** Verfahren nach Anspruch 3, wobei der Katalysator Zirkonoxid und mindestens ein metallisches Element aus der Gruppe Chrom, Mangan, Kobalt, Zink, Indium und Blei umfaßt.

**8.** Verfahren nach Anspruch 3, wobei der Katalysator Zirkonoxid und Chrom umfaßt.

**9.** Verfahren nach Anspruch 6, wobei das metallische Element in einem Atomverhältnis von 0.001 bis 0.5, bezogen auf Zirkon, vorhanden ist.

**10.** Verfahren nach Anspruch 1, wobei die Hydrierung bei einer Temperatur von 260 bis 400 °C durchgeführt wird, während die heterocyclische Carbonsäure oder das Derivat davon mit einer Raumgeschwindigkeit der Flüssigkeit von 0.01 bis 1 hr$^{-1}$ und Wasserstoff mit einer Raumgeschwindigkeit des Gases von 100 bis 20000 hr$^{-1}$ eingespeist werden.

**Revendications**

**1.** Procédé de production d'un aldéhyde hétérocyclique, qui comprend l'hydrogénation d'un acide carboxylique hétérocyclique contenant au moins un hétéro atome choisi parmi les atomes d'azote, de soufre et d'oxygène, comme élément constitutif d'un noyau hétérocyclique, ou d'un dérivé de celui-ci, par l'hydrogène moléculaire, en présence d'un catalyseur contenant un oxyde d au moins un élément choisi dans le groupe constitué du zinc, de l'yttrium, du cérium, du titane, du zirconium, et du hafnium.

**2.** Procédé selon la revendication 1, dans lequel ledit acide carboxylique hétérocyclique ou son dérivé contient un noyau pyrrole, furanne, thiophène, thiazole, pyrane ou pyridine.

**3.** Procédé selon la revendication 1, dans lequel ledit acide carboxylique hétérocyclique ou son dérivé est l'acide nicotinique, l'acide furane-carboxylique ou l'acide thiazole-carboxylique ou un dérivé de ceux-ci.

**4.** Procédé selon la revendication 3, dans lequel ledit dérivé de l'acide carboxylique hétérocyclique est un ester comportant un radical alkyle ayant de 1 à 4 atomes de carbone, un radical phényle ou cyclohexyle.

**5.** Procédé selon la revendication 1, dans lequel ledit catalyseur contient de l'oxyde de zirconium, de l'oxyde de hafnium, de l'oxyde de titane, de l'oxyde d'yttrium, de l'oxyde de zinc ou de l'oxyde de cérium.

**6.** Procédé selon la revendication 1, dans lequel ledit catalyseur comprend de l'oxyde de zirconium et au moins un élément métallique choisi dans le groupe constitué du chrome, du manganèse, du cobalt, du zinc, de l'indium et du plomb.

**7.** Procédé selon la revendication 3, dans lequel ledit catalyseur comprend de l'oxyde de zirconium et au moins un élément métallique choisi dans le groupe constitué du chrome, du manganèse, du cobalt, du zinc, de l'indium, et du plomb.

**8.** Procédé selon la revendication 3, dans lequel ledit catalyseur comprend de l'oxyde de zirconium et du chrome.

**9.** Procédé selon la revendication 6, dans lequel ledit élément métallique est présent dans un rapport atomique de 0,001 à 0,5 par rapport au zirconium.

**10.** Procédé selon la revendication 1, dans lequel ladite hydrogénation est effectuée à une température de 260 à 400 °C, en introduisant l'acide carboxylique hétérocyclique ou son dérivé à un débit horaire de liquide de 0,01 à 1 h$^{-1}$, et l'hydrogène à un débit horaire de gaz de 100 à 20.000 h$^{-1}$.